# EUROPEAN PATENT APPLICATION

(11) **EP 2 058 009 A1**
(43) Date of publication of application: **13.05.2009**
(21) Application number: 07806890.5
(22) Date of filing: 31.08.2007
(51) Int. Cl.: A61K 45/00, A61K 9/127, A61K 39/385, A61P 37/02, A61P 37/06, A61P 37/08, A61P 43/00

(54) **REVERSE TARGETING LIPID VESICLE**

(30) Priority: 31.08.2006 JP 2006235375
(71) Applicant: Astellas Pharma Inc., Tokyo 103-8411 (JP); Oku, Naoto, Shimizu-ku Shizuoka-shi Shizuoka 424-0857 (JP)
(72) Inventor: OKU, Naoto, Shizuoka-shi Shizuoka 424-0857 (JP); ASAI, Tomohiro, Shizuoka-shi Shizuoka; 424-0857 (JP); URAKAMI, Takeo, Orlando, FL 32832 (US)
(74) Representative: Denison, Christopher Marcus
(86) International application number: PCT/JP2007/067449
(87) International publication number: WO 2008/026781

(57) **Abstract**

The present invention provides an immunomodulating agent and a therapeutic agent for a immune disease, which comprise a lipid vesicle encapsulating a drug capable of suppressing or damaging an immunocyte that expresses or binds to an antibody, the lipid vesicle having an antigen for the antibody bound to the outer surface thereof. Hence, there is provided means for effectively treating an immune disease such as an allergic diseases or an autoimmune disease.

## Description

### FIELD OF THE INVENTION

The present invention relates to a lipid vesicle useful for immunomodulation and treatment of an immune disease.

### BACKGROUND OF THE INVENTION

Conventionally, a drug delivery system (DDS) for delivering a drug administered into the blood to the target site has been known, for example, as a DDS formulation of a type that causes passive accumulation of the drug carrier in the tumor site by exploiting enhanced vascular permeability at the tumor (passive targeting) ^{(1),(2)}. Since this type of DDS formulation has been poor in intracellular drug incorporation efficiency, a DDS formulation of a type that causes active accumulation of the drug carrier in the target tissue including tumor sites (active targeting) has been developed as the next-generation DDS, which has been reported effective also in terms of drug incorporation efficiency ⁽³⁾.

On the other hand, an antibody molecule has practically been used for antibody therapy in diverse ways by exploiting its high affinity for an antigen as a target substance, and is noted for its use as a targeting probe for DDS.

Therapeutic methods known for so-called immune diseases such as allergic diseases and autoimmune diseases include methods for diminishing the symptoms by administering a steroidal or nonsteroidal anti-inflammatory drug, an inhibitor for a chemical mediator receptor such as histamine released from mast cells or a release suppressor for histamine from mast cells or the like, and a method for suppressing autoimmune reaction by suppressing the immune system with an immunosuppressive agent.

According to the conventional methods, however, sufficient relief of allergic symptoms is difficult and likely to cause various infectious diseases due to suppression of the immune system. Thus, development of essential therapeutic means and method for immune diseases has been desired.
(1) Oku, N., et al., Oncogene, 21, 2662-2669, 2002
(2) Shimizu, K., et al., Expert Opin. Ther. Targets, 9, 63-76, 2005
(3) Ichikawa, K., et al., Biochim. Biophys. Acta, 1669, 69-74, 2005

### DISCLOSURE OF THE INVENTION

A problem to be solved by the invention is to provide means for modulating the immune level in a simple and effective manner and means for treating an immune disease such as an allergic disease or an autoimmune disease in an effective manner.

In order to solve the above problems, we have gone through keen examination, as a result of which we found that an immunocyte can be damaged with a liposome having an antigen for an antibody causing the immune disease bound thereto so as to target the immunocyte that expresses or binds to that antibody, thereby achieving the present invention.

Thus, the present invention relates to the followings.
(1) An immunomodulating agent comprising a lipid vesicle encapsulating a drug capable of suppressing or damaging an immunocyte expressing or binding to an antibody, the lipid vesicle having an antigen for that antibody bound to the outer surface thereof.
   With respect to the immunomodulating agent of the present invention, immunomodulation refers to, for example, immunosuppression.
(2) A therapeutic agent for an immune disease comprising a lipid vesicle encapsulating a drug capable of suppressing or damaging an immunocyte expressing or binding to an antibody, the lipid vesicle having an antigen for that antibody bound to the outer surface thereof.
   With respect to the therapeutic agent of the present invention, an immune disease refers to, for example, an allergic disease or an autoimmune disease.
(3) A method for modulating immunity comprising administering a lipid vesicle encapsulating a drug capable of suppressing or damaging an immunocyte expressing or binding to an antibody, the lipid vesicle having an antigen for that antibody bound to the outer surface thereof.
(4) A method for treating an immune disease comprising administering a lipid vesicle encapsulating a drug capable of suppressing or damaging an immunocyte expressing or binding to an antibody, the lipid vesicle having an antigen for that antibody bound to the outer surface thereof.
(5) A lipid vesicle encapsulating a drug capable of suppressing or damaging an immunocyte expressing or binding to an antibody, the lipid vesicle having an antigen for that antibody bound to the outer surface thereof.

As means for modulating the immune level in a simple and effective manner and as means for treating an immune disease such as an allergic disease or an autoimmune disease in an effective manner, the present invention can provide an immunomodulating agent and a therapeutic agent for an immune disease, comprising a lipid vesicle that binds to an antigen-specific immunocyte to suppress or damage the immunocyte so that the activity of the immunocyte can be suppressed or inhibited. The present invention can also provide a method for modulating immunity and a method for treating an immune disease, comprising administering a lipid vesicle that binds to an antigen-specific immunocyte to suppress or damage the immunocyte so that the activity of the immunocyte can be suppressed or inhibited.

The modulating agent, the therapeutic agent, the method for modulating immunity and the method for treating an immune disease according to the present invention are remarkably useful in that they are capable of realizing essential treatment of an immune disease that has previously been difficult and in that they are highly effective.

### BRIEF DESCRIPTION OF THE DRAWINGS

Figure 1 shows graphs showing the presence or the absence of a side effect (weight loss) caused by drug-encapsulating liposomes (A and B).
Figure 2 shows graphs showing the influences of administration of various liposomes on IgG production (A) and IgE production (B).
Figure 3 shows graphs showing the influences of administration of various liposomes on IgE production. (A) Day 22 and (B) Day 36.

### BEST MODES FOR CARRYING OUT THE INVENTION

Hereinafter, the present invention will be described in detail. The scope of the invention should not be limited to the following description and the present invention may be modified suitably apart from the following examples without departing from the spirit of the invention.

### 1. Summary of the Invention

By employing the completely opposite idea to the targeting DDS that lets a drug carrier recognize and access a tissue or a cell of interest, the present invention proposes and demonstrates an intelligent reverse targeting DDS (RT-DDS) that lets the cell of interest recognize the drug carrier, thereby providing, in particular, means for immunomodulation and treatment of an immune disease.

Specifically, the present invention is based on an idea of reversing antigen-antibody reaction, where an antigen-modified (antigen-bound) DDS formulation is used to reversely target an immunocyte (an antibody-expressing cell or an antibody-bound cell) so as to specifically damage the immunocyte, thereby providing means for modulating the immune level in a simple and effective manner or means for essentially treating an allergic disease or an autoimmune disease.

To be precise, a lipid vesicle such as a liposome used in the invention encapsulates in its internal water or lipid phase a drug capable of damaging an immunocyte that expresses or binds to the antibody, and has an antigen for the antibody bound to the outer surface thereof. This lipid vesicle may be used as an immunomodulating agent or a therapeutic agent for an immune disease.

The phrase "capable of suppressing or damaging an immunocyte" refers to suppressing or inhibiting the function or the activity of the immunocyte as well as weakening or eliminating homeostasis of the immunocyte, which includes being capable of destructing cell morphology and inducing cell death such as apoptosis.

The immunomodulating agent is administered as an injection or in other dosage forms such eye drops, nasal drops, aerosols, suppository, liniment, adhesive skin patch or the like to a patient suffering from an allergic disease or an autoimmune disease in expectation of a therapeutic effect.

The above-mentioned immunocytes may comprise, but not limited to, any cells included in the white blood cells. An example of such immunocytes includes a cell that retains on its outer surface an antibody involved in an immune disease or a molecule similar to such an antibody in that it recognizes the antigen. Preferably, such an immunocyte is an antibody-expressing cell or an antibody-bound cell. The term an "antibody-expressing cell" refers to a cell that produces and retains on the outer surface thereof an antibody to a substance (antigen) causing an immune disease such as an allergic disease or an autoimmune disease. An example of such a cell is a B cell. The antibody-expressing cell also comprises a T cell that directly or indirectly recognizes the antigen and thus influences the antibody production of the B cell. The term an "antibody-bound cell" refers to a cell that retains on the outer surface thereof an antibody to a substance (antigen) causing an allergic disease, the antibody being produced by the antibody-expressing cell. Examples of such antibody-bound cells include mast cells bound to the antibody and B cells bound to the antibody.

### 2. Lipid Vesicle Used with the Invention

A lipid vesicle used in an immunomodulating agent or a therapeutic agent for an immune disease of the invention comprises, as constituents, a lipid, a drug to be encapsulated into the lipid vesicle and an antigen that binds to the outer surface of the vesicle.

### (1) Lipid vesicle

One or more types of lipids selected from various types of known amphiphilic molecules that may form a bilayer membrane in an aqueous vehicle may be used as a constituent of the vesicle membrane. Preferably, it is a native or synthetic saturated or unsaturated phospholipid or a combination thereof, more preferably it is a synthetic saturated phospholipid.

Examples of preferable saturated phospholipids include, but not limited to, native phospholipids and derivatives thereof, for example, extracts from outer and inner cell membranes such as hydrogenated egg-yolk lecithin, hydrogenated soybean lecithin, liver plasma membrane, erythrocyte membrane and *E*. *coli* membrane, diacyl phosphatidylcholine such as distearoyl phosphatidylcholine, dimyristoyl phosphatidylcholine and dipalmitoyl phosphatidylcholine, diacylphosphatidic acid, diacylphosphatidylethanolamine, diacylphosphatidylglycerol, diacylphosphatidylinositol, sphingophospholipids and the like, among which dimyristoyl phosphatidylcholine, dipalmitoyl phosphatidylcholine and distearoyl phosphatidylcholine are more preferable.

Examples of preferable unsaturated phospholipids include, but not limited to, native unsaturated phospholipids such as egg-yolk lecithin and soybean lecithin, synthetic unsaturated phospholipids such as 1-palmitoyl,2-oleoylphosphatidylcholine, dioleoylphosphatidylcholine and dilinoleoylphosphatidylcholine, polymerizable phospholipids such as 1,2-di(octadeca-trans-2,trans-4-dienoyl)phosphatidylcholine and 1,2-bis-eleostearoylphosphatidylcholine. The polymerizable phospholipids mentioned above may have a non-polymerizable long chain such as, but not limited to, a linear or branched-chain alkyl group, acyl group, non-polymerizable alkenyl group and a non-polymerizable alkenoyl group having a carbon number of 2 to 24.

Examples of a combination of a saturated phospholipid and an unsaturated phospholipid include, but not limited to, a combination of a hydrogenated egg-yolk phospholipid and an egg-yolk phospholipid, a combination of a synthetic saturated phospholipid such as dipalmitoyl phosphatidylcholine or distearoyl phosphatidylcholine and a synthetic unsaturated phospholipid such as 1-palmitoyl,2-oleoylphosphatidylcholine or dioleoylphosphatidylcholine, among which a combination of dipalmitoyl phosphatidylcholine and 1-palmitoyl,2-oleoylphosphatidylcholine is preferable.

Preferably, the lipid constituting the membrane of the vesicle comprises a polyethylene glycol (PEG) lipid, namely a lipid having a PEG chain bound thereto. Inclusion of a PEG lipid will form a lipid vesicle that has a PEG chain on the surface thereof. Hence, variation of the particle sizes of the vesicles due to aggregation or fusion of the vesicles can be controlled effectively when the dried vesicle is redispersed in an aqueous solution or during storage, and moreover deterioration of permeability or clogging of the filter upon extrusion for controlling the particle size can be prevented. Furthermore, blood dynamics in the blood can be improved effectively.

When the vesicle includes a PEG lipid, the percentage thereof is, but not limited to, preferably 1-20 mol%, and more preferably 2-10 mol% to the whole membrane-constituting lipid. The molecular weight of the PEG chain in the PEG lipid is, but not limited to, for example, 500-12,000, and more preferably 1,500-5,000. If the molecular weight of the PEG chain is within the range mentioned above, the variation of the particle sizes or the aggregation mentioned above will be controlled more effectively.

The membrane-constituting lipid of the vesicle may include a lipid having a sugar chain such as a polysaccharide bound thereto. When such a lipid is included, the percentage thereof is, but not limited to, preferably 1-50 mol%, and more preferably 5-20 mol% to the whole membrane-constituting lipid.

The membrane-constituting lipid of the vesicle may include a negatively charged lipid (anionic lipid). Examples of a negatively charged lipid include, but not limited to, diacylphosphatidylglycerol, diacylphosphatidic acid, diacylphosphatidylinositol, diacylphosphatidylserine and fatty acids. Examples of fatty acids include, but not limited to, saturated or unsaturated fatty acids having a carbon number of 12-20, specifically, myristic acid, palmitic acid, stearic acid, oleic acid, linoleic acid, linolenic acid and octadeca-2,4-dienoic acid.

When the vesicle includes a negatively charged lipid, the percentage thereof is, but not limited to, preferably 1-50 mol%, and more preferably 5-20 mol% to the whole membrane-constituting lipid.

Moreover, the membrane-constituting lipid of the vesicle may include a lipid component as a stabilizer. Examples of such a stabilizer include, but not limited to, sterols. Specific examples include any steroid derivatives having the perhydrocyclopentanophenanthrene skeleton, such as cholesterol, ergosterol, bile acid, provitamin D and steroid hormone, among which cholesterol is preferable.

When the vesicle includes such a lipid component as a stabilizer, the percentage thereof for effectively stabilizing the vesicle membrane is, but not limited to, 5-50 mol%, and preferably 20-33.3 mol% to the whole membrane-constituting lipid.

### (2) Drug

A drug to be encapsulated into the lipid vesicle is not limited as long as it is capable of suppressing or damaging immunocytes. For example, a drug capable of inducing cell death such as apoptosis in various cells is preferable.

When suppression of or damage to an immunocyte is aimed at inducing immunosuppression, the drug may be any known drug which is not particularly limited as long as it is capable of suppressing or damaging the immunocyte. A drug containing a nucleic acid drug or a biological drug may be employed.

Examples of such drugs capable of suppressing or damaging an immunocyte include adriamycin, daunomycin, antilymphocyte antibody, glucocorticoids, 6-mercaptopurine, azathioprine, cyclophosphamide, cyclosporine, tacrolimus (FK506), deoxyspergualin, other immunosuppressive drugs, cytotoxic proteins Fas-L and TNF-α, various nucleic acid drugs, anticancer drugs and antibiotics, where one or more of them may be used. Such drugs, however, are not limited to the drugs enumerated above.

A drug with higher water solubility is generally encapsulated as an aqueous solution in the lipid vesicle. Examples of preferable aqueous vehicles used for this aqueous solution include, but not limited to, those having biocompatibility at least in the blood and capable of retaining the stable state of the encapsulated drug, including, besides water, a pH buffer and a physiological saline. Examples of pH buffers include a glycine buffer, a monopotassium phthalate buffer, a succinate buffer and a monopotassium citrate buffer.

The drug concentration in the aqueous solution above (drug concentration in the internal water phase of the lipid vesicle) is not limited and may be determined appropriately considering the pathological condition of the immune disease or the like.

Tacrolimus used with the invention is a substance produced by genus *Streptomyces* such as *Streptomyces tsukubaensis*, No. 9993 (depository institute: the National Institute of Bioscience and Human-Technology, Agency of Industrial Science and Technology, MITI (former Fermentation Research Institute, Agency of Industrial Science and Technology, MITI), 1-1-3 Higasi, Tsukuba-shi, Ibaragi-ken, Japan, deposit date: October 5, 1984, deposit number: FERM BP-927), *Streptomyces hygroscopicus* subsp. *yakushimaensis*, No. 7238 (depository institute: the National Institute of Bioscience and Human-Technology, Agency of Industrial Science and Technology, MITI, 1-1-3 Higasi, Tsukuba-shi, Ibaragi-ken, Japan, deposit date: January 12, 1985, deposit number: FERM BP-928) (EP-A-0184162) or the like.

The structural formula and the IUPAC name of Tacrolimus used with the invention are as follows.

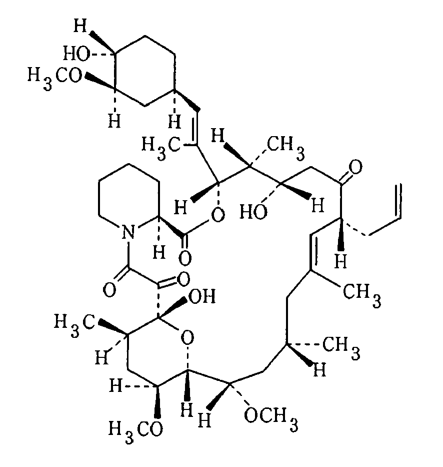

IUPAC name: (-)-(1R, 9S, 12S, 13R, 14S, 17R, 18E, 21S, 23S, 24R, 25S, 27R)-17-allyl-1,14-dihydroxy-12-[(E)-2-[(1R, 3R, 4R)-4-hydroxy-3-methoxycyclohexyl]-1-methylvinyl]-22,25-dimethoxy-13,19,21,27-tetramethyl-11,28-dioxa-4-azatricyclo[22.3.1.0^{4.9}]octacos-18-ene-2,3,10,16-tetraone.

Tacrolimus used with the invention may be a solvate. Examples of solvates include hydrates, alcoholates (e.g., ethanolate) and acetone solvates.

Herein, Tacrolimus used with the invention is useful for treating or preventing the following diseases:
eye diseases due to autoimmune diseases (e.g., keratoconjunctivitis, springtime conjunctivitis, uveitis associated with Behcet's disease, keratitis, herpetic keratitis, conical keratitis, corneal epithelial dystrophy, keratoleukoma, ocular premphigus, Mooren's ulcer, scleritis, Graves' ophthalmopathy, Vogt-Koyanagi-Harada syndrome, keratoconjunctivitis sicca (dry eye), phlyctenule, iridocyclitis, sarcoidosis, endocrine ophthalmopathy, etc.);
skin diseases (e.g., dermatomyositis, vitiligo vulgaris, ichthyosis vulgaris, photosensitivity and cutaneous T-cell lymphoma, inflammatory or hyperproliferative skin diseases and immunologically-mediated skin diseases (specifically, atopic dermatitis, contact dermatitis, eczematoid dermatitis, seborrheic dermatitis, lichen planus, pemphigus, bullous pemphigoid, epidermolysis bullosa, urticaria, angioedema, vasculitides, erythema, dermal eosinophilia, lupus erythematosus, acne, alopecia areata, etc.), psoriasis such as psoriasis arthropica, psoriasis circinata, psoriasis diffusa, psoriasis discoidea, generalized von Zumbusch-type pustular psoriasis, psoriasis geographica, psoriasis guttata, psoriasis gyrata, old psoriasis, psoriasis nummularis, psoriasis anularis, ostraceous psoriasis, psoriasis punctata, pustular psoriasis, psoriasis spondylitis, generalized psoriasis, etc.);
hypertropic cicatrix and keloid due to trauma, burn, surgery or the like;
rejection reactions due to transplantation of organs or tissues such as the heart, kidney, liver, bone marrow, skin, cornea, lung, pancreas, small intestine, limb, muscle, nerve, intervertebral disc, trachea, myoblast, cartilage, etc.;
graft-versus-host reactions following bone marrow transplantation;
rheumatoid arthritis, systemic lupus erythematosus, Hashimoto's thyroiditis, multiple sclerosis, myasthenia gravis, type I diabetes, etc.;
infections caused by pathogenic microorganisms (e.g., *Aspergillus fumigatus*, *Fusarium oxysporum*, *Trichophyton asteroides*, etc.);
reversible obstructive airway diseases [asthma (e.g., bronchial asthma, allergic asthma, intrinsic asthma, extrinsic asthma and dust asthma), particularly chronic or inveterate asthma (e.g., late asthma and bronchitis associated with airway hyperresponsiveness), etc.];
mucosal or vascular inflammations (e.g., gastric ulcer, ischemic or thrombotic vascular injury, ischemic bowel diseases, enteritis, necrotizing enterocolitis, intestinal damages associated with thermal bums and leukotriene B4-mediated diseases);
intestinal inflammations/allergies (e.g., coeliac diseases, proctitis, eosinophilic gastroenteritis, mastocytosis, Crohn's disease and ulcerative colitis);
food-related allergic diseases with symptomatic manifestation remote from the gastrointestinal tract (e.g., migraine, rhinitis and eczema);
renal diseases (e.g., interstitial nephritis, Goodpasture's syndrome, hemolytic uremic syndrome and diabetic kidney disorder);
nervous diseases (e.g., multiple myositis, Guillain-Barre syndrome, Meniere's disease, multiple neuritis, solitary neuritis, cerebral infarctions, Alzheimer's disease, Parkinson's disease, amyotrophic lateral sclerosis (ALS) and radiculopathy);
cerebral ischemic diseases (e.g., head injury, hemorrhage in brain (e.g., subarachnoid hemorrhage, intracerebral hemorrhage), cerebral thrombosis, cerebral embolism, cardiac arrest, stroke, transient ischemic attack (TIA), hypertensive encephalopathy and cerebral infarction) ;
endocrine diseases (e.g., hyperthyroidism and Basedow's disease);
hematic diseases (e.g., pure red cell aplasia, aplastic anemia, hypoplastic anemia, idiopathic thrombocytopenic purpura, autoimmune hemolytic anemia, agranulocytosis, malignant anemia, megaloblastic anemia and anerythroplasia);
bone diseases (e.g., osteoporosis);
respiratory diseases (e.g., sarcoidosis, pulmonary fibrosis, and idiopathic interstitial pneumonia);
skin diseases (e.g., dermatomyositis, vitiligo vulgaris, ichthyosis vulgaris, photosensitivity and cutaneous T-cell lymphoma);
circulatory diseases (e.g., arteriosclerosis, atherosclerosis, aortitis syndrome and multiple arteritis);
collagen diseases (e.g., scleroderma, Wegener's granuloma and Sjogren's syndrome);
obesity;
eosinophilic fasciitis;
periodontal diseases (e.g., damage to gingiva, periodontium, alveolar bone and substantia ossea dentis);
nephrotic syndrome (e.g., kidney inflammation);
male pattern alopecia, alopecia senile;
muscular dystrophy;
pyoderma and Sezary syndrome;
chromosome abnormality-associated diseases (e.g., Down's syndrome); and
Addison's disease.
(see Japanese Laid-Open Patent Publication No. 61-148181, European Patent Publication Nos. 0184162 and 0323042, etc.).

Although the amount of tacrolimus used with the invention is not particularly limited as long as it is adjusted appropriately in terms of treatment or prevention according to the age of each patient, the type of the disease, the degree of the disease or other factors, it is generally about 0.05-500 mg, preferably 0.1-100 mg per day. The given dose at a time is generally 0.1 mg, 0.5 mg, 1 mg, 5mg, 10 mg, 50 mg or 100 mg in average (which is indicated as an amount of tacrolimus when provided in a form of a solvate such as a hydrate). In general, the number of doses is twice a day.

### (3) Antigen

An antigen bound to the outer surface of the lipid vesicle is not particularly limited as long as it is a substance capable of binding to an immunocyte to be damaged by the above-mentioned drug (specifically, a receptor or an antibody existing on the surface of the immunocyte). For example, it is preferably a causative substance of an immune disease associated with the immunocyte. The causative substance may be a substance having an allergen or an epitope identical thereto in the case of an allergic disease, or a substance having an autoantigen or an epitope identical thereto in the case of an autoimmune disease.

In the lipid vesicle used with the invention, an embodiment where an antigen is bound to the outer surface of the lipid vesicle is not limited to an embodiment where an antigen is directly bound to a lipid molecular constituting the lipid vesicle, and also comprises an embodiment where the antigen is indirectly bound to the lipid molecular via other substance that is directly bound to the lipid molecule. This indirect binding comprises an embodiment where the antigen is bound to a PEG chain bound to a lipid molecular constituting the lipid vesicle.

### (4) Other components

Apart from the above-mentioned lipid vesicle, a drug included in the vesicle and an antigen bound to the outer surface of the vesicle, the lipid vesicle used with the invention may suitably comprise other components. Examples of other components to be contained in the inter water phase of the lipid vesicle include amino acids, reducing agents, vitamins, bases and the like.

Furthermore, other than an antigen, the surface of the lipid vesicle may appropriately include a substance for modifying the surface of the lipid vesicle for the purpose of improving the therapeutic efficacy. Examples of the surface-modifying substances include proteins, peptides, sugar chains, synthetic high-molecular compounds and synthetic low-molecular compounds that are capable of enhancing affinity with a target cell.

### (5) Method for producing lipid vesicle

The method for producing a lipid vesicle used with the invention is not particularly limited and any method may be employed. In general, a vesicle made of a lipid bilayer membrane is prepared and then a drug is encapsulated in the vesicle at the same time or after the preparation. Then, an antigen is bound to the outer surface of the vesicle or an antigen-bound lipid molecule is reacted with the heterogeneous vesicle. This will be specifically described hereinafter.

For example, as a method for encapsulating a drug in a vesicle made of a lipid bilayer membrane, one may employ a method comprising preparing a lipid vesicle, and then encapsulating a drug (aqueous drug solution) in the internal water phase or the lipid phase of this vesicle. A preferable example of such a method includes a remote loading method (see "N. Oku, K. Doi, Y. Namba, S. Okada, Therapeutic effect of adriamycin encapsulated in long-circulating liposomes on Meth-A-Sarcoma-bearing mice, International Journal of Cancer, 58(3), 415-419, 1994").

Other methods that may be employed for encapsulating a drug in a lipid vesicle are the known methods generally used for preparing a lipid vesicle. For example, vortex, ultrasound irradiation, high pressure pumping, high pressure extrusion, forced agitation, freezing-and-thawing, organic solvent injection, surfactant removal, reversed-phase evaporation or the like may appropriately be selected or appropriately be combined with one another. Means and conditions for carrying out each method may suitably be determined following common knowledge of those skilled in the art. Specifically, the intended membrane-forming lipid (generally in a powdered form) is added to an aqueous solution of a drug to be encapsulated so that the lipid is hydrated and swollen. The resultant is left to stand, and the lipid is dispersed, for example, with a vortex mixer, a forced agitator, an ultrasound irradiator (e.g., homogenizer), a microfluidizer, a high pressure extruder or by freezing-and-thawing, thereby obtaining dispersion of the lipid vesicle containing the aqueous drug solution (i.e., encapsulating the drug) as the internal water phase.

In general, the particle size of the eventual lipid vesicle often turns out to be smaller than that obtained at the stage of encapsulating the drug. Hence, the size is prospectively controlled in anticipation of such reduction preferably in terms of productivity. Although the particle size (number average particle size) of the eventual lipid vesicle is not limited, in the case of systemic administration, it is preferably 50-400 nm, more preferably 80-200 nm for obtaining better results for encapsulation efficiency, sterilization, blood kinetics and the like. For local administration, however, the size is not limited thereto and even a size of about 5 µm is acceptable.

Subsequently, an antigen is bound to the outer surface of the drug-encapsulating lipid vesicle. A method for binding an antigen to the outer surface of a lipid vesicle is not limited and it may be, for example, a method in which an antigen is added to a dispersant containing the lipid vesicle for direct reaction or a method where a lipid molecule having an antigen bound directly or indirectly thereto is prepared beforehand and then added to a dispersant containing the lipid vesicle for reaction. By following the above-mentioned procedure, a drug-encapsulating lipid vesicle having an antigen bound to the outer surface thereof may be obtained.

Preferably, where necessary, unencapsulated drugs and unreacted antigens or unreacted lipid molecules are separated and removed afterward, for example, by gel filtration chromatography, centrifugation (ultracentrifugation), ultrafiltration membrane treatment or the like.

### (6) Usefulness of lipid vesicle

Since a lipid vesicle used with the invention has structural components and a configuration described above, it serves as an active element of a so-called reverse targeting DDS formulation capable of binding to antibodies or receptors of various immunocytes involved in immune diseases through the bloodstream to give damage to the immunocytes with a drug, thereby suppressing or inhibiting the activity thereof. Accordingly, as will be described below, it is highly useful as an active element of an immunomodulating agent or a therapeutic agent for various immune diseases such as allergic diseases and autoimmune diseases.

### 3. Immunomodulating Agent and Therapeutic Agent for Immune Disease

An immunomodulating agent or a therapeutic agent for an immune disease according to the present invention is characterized by comprising a lipid vesicle described in section 2 above. The present invention also comprises use of the lipid vesicle described in section 2 above for producing the immunomodulating agent or for producing the therapeutic agent for various immune diseases such as allergic diseases and autoimmune diseases. Herein, immunomodulation mentioned above is preferably immunosuppression. The immune diseases mentioned above may be, but not limited to, congenital or acquired.

In general, the immunomodulating agent or the therapeutic agent of the invention is preferably the lipid vesicle described in section 2 above dispersed in a suitable dispersion vehicle (dispersant) so as to allow intravenous infusion or the like upon administration to a living organism. Examples of dispersion vehicles include, but not limited to, pure water, aqueous solutions and buffer solutions, which may be selected appropriately according to usage, type of the disease or the like. In general, injection solvents and physiological salines are preferable in terms of safety in a living organism. The pH of the dispersion vehicle is not limited as long as it is within a physiological pH range, and may be determined suitably within a range that does not have a significant adverse effect such as deterioration of the stability or change in the aggregate morphology of the lipid vesicles. A physiological pH range refers to a pH range in a living organism, blood or the like, which is, for example, pH 4-11, preferably pH 7-8 (generally neutral).

For the modulating agent or the therapeutic agent of the invention, the percentage of the lipid vesicle contained in the dispersion vehicle may be determined appropriately according to the type and the progress of the disease, condition of the patient, the type of the drug to be encapsulated in the vesicle and the like.

A modulating agent or a therapeutic agent of the invention may contain components other than the lipid vesicle and a dispersion vehicle. Examples of other components include excipients, surfactants, dispersants, buffers, preservatives, solubilizing agents, antiseptics, flavoring agents, soothing agents, stabilizers and tonicity agents that are generally used for producing a drug, and further in the case where an injection product is produced, aliphatic polyalcohols such as glycerol, propylene glycol and polyethyleneglycol, which may be added according to a routine method for preparation.

When the modulating agent or the therapeutic agent of the invention is used as a parenteral agent such as an injectable solution, the form thereof is usually not limited, and it may be, for example, an intravenously injectable solution (including intravenous drip), an intramuscularly injectable solution, an intraperitoneally injectable solution or a subcutaneously injectable solution. When used as an injectable solution, the agent of the invention is may be provided in a unit-dose ampoule or a multiple-dose vial, or as lyophilized powder that may be redissolved in a dissolving solution upon use.

### 4. Method for Modulating Immune and Method for Treating Immune Disease

The present invention comprises a method for modulating immune or a method for treating an immune disease, the method comprising administering the modulating agent or the therapeutic agent for an immune disease of the invention to a patient suffering from an immune disease such as an allergic disease or an autoimmune disease.

According to the present invention, although the method for administering the agent to a patient is not particularly limited and any method may be employed, parenteral usage, for example, injectable solutions such as intravenous injection (including intravenous drip), intramuscular injection, intraperitoneal injection and subcutaneous injection as well as eye drops, nasal drops, aerosols, suppository, liniment, adhesive skin patch and the like are preferable.

The given dose, administration time, administration interval and number of administration (per day) may be appropriately determined considering the age, weight and condition of the administration target (patient) and the type and progress level of the disease in addition to the proportion of the active element (lipid vesicle and the drug encapsulated therein) in the formulation to be administered.

The immunomodulating agent or the therapeutic agent for an immune disease of the invention may be administered to mammals other than human in need of the immunomodulation or the treatment of an immune disease. Examples of mammals other than human as the administration target include, but not limited to, livestocks such as cow, horse, sheep and goat, pet animals such as dog and cat, and experimental animals such as mouse, rat, guinea pig and rabbit.

Hereinafter, the present invention will be described more specifically by means of examples, although the present invention should not be limited thereto.

### [EXAMPLE 1]

### <Immunosuppression in ovalbumin (OVA)-hypersensitive mouse models>

### 1. Summary

In this example, IgG-producing and IgE-producing ovalbumin (OVA)-sensitive mice were used as a model system, to which OVA-conjugated intelligent liposomes were administered for recognition by the antibody-producing cells or the antibody-binding mast cells so that these cells were damaged by the drug (adriamycin) encapsulated in the liposomes to see if this causes decrease in IgG and IgE productions.

As a result, administration of the OVA-conjugated liposome encapsulating the drug significantly decreased IgG and IgE productions. These results demonstrate usefulness of immunomodulation and treatment of an immune disease by RT-DDS using the modulating agent or the therapeutic agent of the invention.

### 2. Method and Results

### (1) Preparation of liposomes

Liposomes were prepared by a thin layer method. Specifically, distearoyl phosphatidylcholine (Nippon Fine Chemical) and cholesterol (Sigma Aldrich Japan) were mixed in a molar ratio of 2:1, which was then hydrated with a citrate buffer to prepare a liposome made of a lipid bilayer membrane. The liposomes were passed through a 100-nm-pored polycarbonate membrane to regulate the particle size to be about 100-200 nm.

Then, adriamycin as a drug was encapsulated in the prepared liposome. Adriamycin was encapsulated in the liposome by a remote loading method (see "N. Oku, K. Doi, Y. Namba, S. Okada, Therapeutic effect of adriamycin encapsulated in long-circulating liposomes on Meth-A-Sarcoma-bearing mice, International Journal of Cancer, 58(3), 415-419, 1994"). The amount of adriamycin encapsulated in the liposome was about 1 µg per 1 x 10¹¹ liposomes (total amount of distearoyl phosphatidylcholine and cholesterol: 3.9 µg).

### (2) OVA modification of outer surface of liposome

33.2 mg of OVA (Sigma Aldrich Japan) was dissolved in a borate buffer, with which 0.149 mg of 3-(N-succinimidyloxyglutaryl)aminoprolyl, polyethyleneglycolcarbamyl distearoyl phosphatidylethanolamine (NOF Corporation) was mixed for reaction at 4°C for 18 hours. Thereafter, the reaction solution was mixed with the previously prepared liposome solution for reaction at 65°C for 15 minutes. By going through these reactions, the outer surface of the liposome was OVA-modified (bound with OVA). Then, unreacted substances were removed by sepharose CL-4B column liquid chromatography (GE Healthcare Bio-Sciences).

OVA bound to the outer surface of the liposome was quantified by high-performance liquid chromatography (Shimadzu Corporation) with Column G3000SW (Tosoh Corporation) following solubilization with n-octyl-β-D-glucoside (Dojindo Laboratories). The amount of OVA bound to the outer surface of the liposome was about 500 per liposome (total amount of distearoyl phosphatidylcholine and cholesterol: 3.9 x 10⁻¹⁶ g)_{.}

Thus, a liposome encapsulating adriamycin and having the outer surface modified with OVA (OVA-ADM) was prepared.

An adriamycin-encapsulating liposome (no OVA modification, Lipo-ADM) and an OVA-modified liposome (no encapsulation of adriamycin, OVA-Lipo) were prepared as well, as controls.

### (3) Preparation of OVA-hypersensitive mouse models

A mixture of 100 µg/0.05 mL OVA and 0.05 mL Imject^{™} Alum (PIERCE) per mouse was intraperitoneally injected to six-week-old Balb/c female mice for sensitization to OVA. On Days 15 and 29 (provided that the day of sensitization was Day 0), booster immunization was performed in the same manner as the primary sensitization.

### (4) Side effects of adriamycin

The presence of weight loss as a side effect of adriamycin encapsulated in the liposome was assessed. Three types of test drugs, namely, 0.3 M glucose (Vehicle) as a control, 10 mg/kg (weight) adriamycin (ADM (10 mg/kg)) and 10 mg/kg (weight) adriamycin-encapsulating liposome (no OVA modification, Lipo-ADM (10 mg/kg)) were used for administration. Each of the drugs was intravenously (tail vein) administered to each mouse group on Days 8, 10 and 12 following the sensitization day (Day 0) (total of three doses). Booster immunization was carried out as described above. The 10 mg/kg (weight) adriamycin is a dose that gives medicinal benefits as a cancer chemotherapeutic agent.

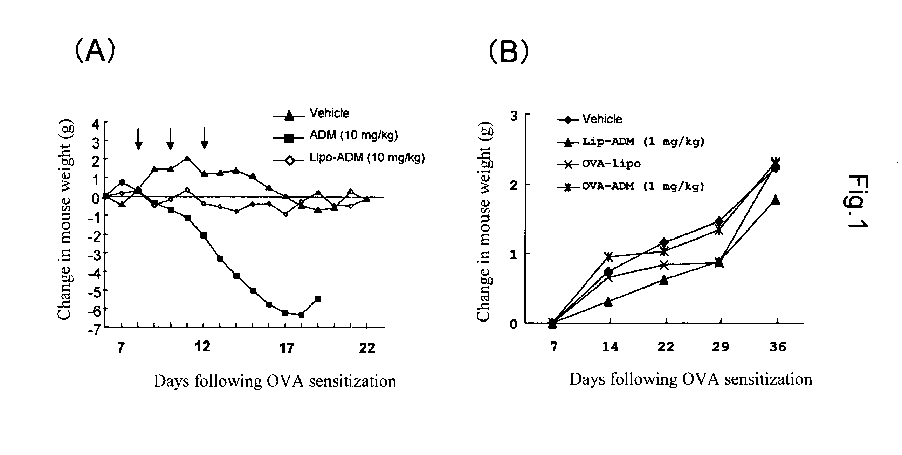

As a result, there was obvious weight loss with adriamycin alone whereas the adriamycin-encapsulating liposome gave no significant difference from Vehicle, showing that the side effect was avoided (see Figure 1(A)).

### (5) Influence on IgG and IgE productions

The OVA-hypersensitive mouse models and the various liposomes described above were used to see if they can significantly decrease the IgG and IgE productions. Meanwhile, weight loss as a side effect was also assessed.

Four types of test drugs, namely, 0.3 M glucose (Vehicle) as a control, 1 mg/kg (weight) adriamycin-encapsulating liposome (no OVA modification, Lipo-ADM (1 mg/kg)), OVA-modified liposome (no encapsulation of adriamycin, OVA-Lipo) and 1 mg/kg (weight) adriamycin-encapsulating OVA-modified liposome (OVA-ADM (1 mg/kg)) were used for administration. Each of the drugs was intravenously (tail vein) administered to each mouse group on Days 8, 10 and 12 following the sensitization day (Day 0) (total of three doses). Booster immunizations were carried out on Days 15 and 29 as described above. Sera were obtained from the mice on Day 36 by drawing blood from the fundus arteries with a hematocrit capillary tube (Terumo Corporation), which was left to stand still and then centrifuged. OVA-specific IgG and IgE in the mouse sera were quantified by ELISA.

As a result, there was no significant difference in the weight loss of the mice between any test drug and Vehicle, indicating avoidance of the side effect (see Figure 1(B)). These seem to result from inclusion of liposomes except for Vehicle and to lower doses of adriamycin.

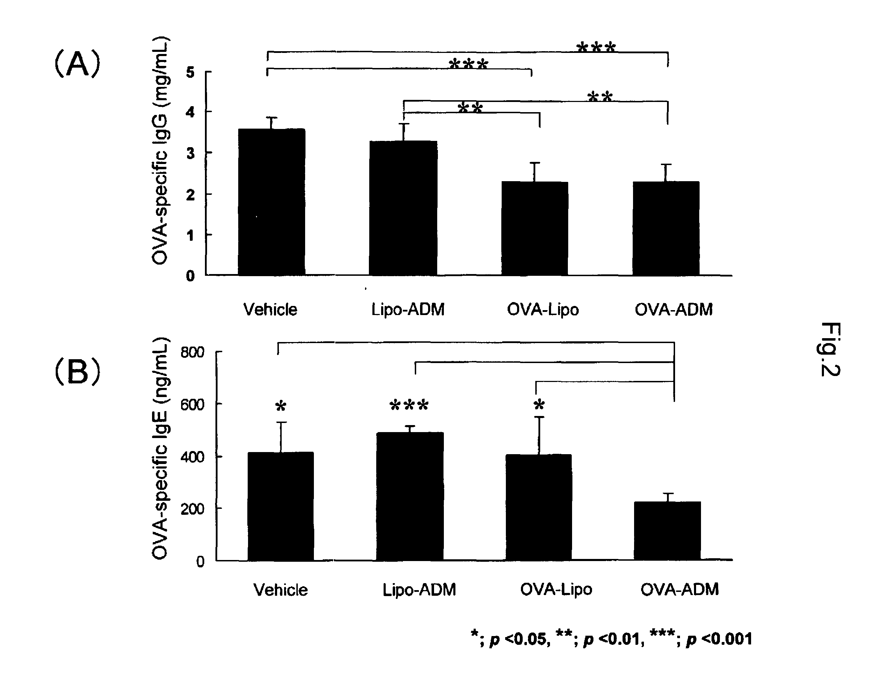

Secondary, as to the influence on production of IgG, the most abundant immunoglobulin in the serum, the administration of OVA-ADM (1 mg/kg) significantly decreased the IgG production as compared to the administrations of Vehicle and Lipo-ADM (1 mg/kg) (see Figure 2(A)). These results indicate that OVA-ADM (1 mg/kg) is directly or indirectly recognized by the immunocompetent cells, causing biological reaction and thus selectively and effectively suppressing IgG production.

Moreover, as to the influence on production of IgE, an immunoglobulin intimately related to various allergic reactions, the administration of OVA-ADM (1 mg/kg) significantly decreased the IgE production as compared to the administrations of Vehicle, Lipo-ADM (1 mg/kg) and OVA-Lipo (see Figure 2(B)). These results indicate that OVA-ADM (1 mg/kg) has an effect of selectively and effectively suppressing IgE production unlike the drug merely encapsulating adriamycin (Lipo-ADM) or the drug merely modified with OVA (OVA-Lipo).

### [EXAMPLE 2]

### <Immunosuppression in ovalbumin (OVA)-hypersensitive mouse models>

### 1. Summary

IgE-producing ovalbumin (OVA)-sensitive mice were used as a model system, to which OVA-conjugated intelligent liposomes were administered for recognition by the antibody-producing cells or the antibody-binding mast cells so that these cells were damaged by the drug (tacrolimus) encapsulated in the liposomes to see if this causes decrease in IgE production.

As a result, administration of the OVA-conjugated liposome encapsulating the drug significantly decreased IgE production. This result demonstrates usefulness of immunomodulation and treatment of an immune disease by RT-DDS using the modulating agent or the therapeutic agent of the invention.

### 2. Method and Results

### (1) Preparation of liposomes

Liposomes were prepared by a freeze-drying process. Specifically, dipalmitoyl phosphatidylcholine, dipalmitoyl phosphatidylglycerol (Nippon Fine Chemical) and tacrolimus were mixed in a molar ratio of 9:1:0.2. The resultant was dissolved in chloroform and a suitable amount of t-butanol. Chloroform was distilled away by evaporation and the resultant was subjected to freezing and drying with liquid nitrogen. An isotonic phosphate buffer was used for hydration to prepare a liposome made of a lipid bilayer membrane. The liposomes were passed through a 100-nm-pored polycarbonate membrane to regulate the particle size to be about 100-200 nm.

### (2) OVA modification of outer surface of liposome

33.2 mg of OVA (Sigma Aldrich Japan) was dissolved in a borate buffer, with which 0.149 mg of 3-(N-succinimidyloxyglutaryl)aminoprolyl, polyethyleneglycol-carbamyl distearoyl phosphatidylethanolamine (NOF Corporation) was mixed for reaction at 4°C for 18 hours. Thereafter, the reaction solution was mixed with the previously prepared liposome solution for reaction at 65°C for 15 minutes. By going through these reactions, the outer surface of the liposome was OVA-modified (bound with OVA). Then, unreacted substances were removed by sepharose CL-4B column liquid chromatography (GE Healthcare Bio-Sciences).

OVA bound to the outer surface of the liposome was quantified by high-performance liquid chromatography (Shimadzu Corporation) with Column G3000SW (Tosoh Corporation) following solubilization with n-octyl-β-D-glucoside (Dojindo Laboratories). The amount of OVA bound to the outer surface of the liposome was about 88 per liposome (total amount of dipalmitoyl phosphatidylcholine and dipalmitoyl phosphatidylglycerol: 1.2 x 10⁻¹⁴ g). Thus, a liposome encapsulating tacrolimus and having the outer surface modified with OVA (OVA-FK) was prepared. A tacrolimus-encapsulating liposome (no OVA modification, FK-Lipo) and an OVA-modified liposome (no encapsulation of tacrolimus, OVA-Lipo) were prepared as well, as controls.

### (3) Preparation of OVA-hypersensitive mouse models

A mixture of 100 µg/0.05 mL OVA and 0.05 mL Imject^{™} Alum (PIERCE) per mouse was intraperitoneally injected to six-week-old Balb/c female mice (Japan SLC, Inc.) for sensitization to OVA. On Days 15 and 29 (provided that the day of sensitization was Day 0), booster immunization was performed in the same manner as the primary sensitization.

### (4) Side effects of tacrolimus

The presence of weight loss as a side effect of tacrolimus encapsulated in the liposome was assessed. Four types of test drugs, namely, 0.3 M glucose (Vehicle) as a control, 5 µg/kg (weight) tacrolimus-encapsulating liposome (no OVA modification, FK-Lipo (5 µg/kg)), OVA-modified liposome (no encapsulation of tacrolimus, OVA-Lipo) and 5 µg/kg (weight) tacrolimus-encapsulating OVA-modified liposome (OVA-FK (5 µg/kg)) were used for administration. Each of the drugs was intravenously (tail vein) administered to each mouse group on Days 8, 10 and 12 following the sensitization day (Day 0) (total of three doses). Booster immunization was carried out as described above.

### (5) Influence on IgE production

The OVA-hypersensitive mouse models and the various liposomes described above were used to see if they can significantly decrease the IgE production. Four types of test drugs, namely, 0.3 M glucose (Vehicle) as a control, 5 µg/kg (weight) tacrolimus-encapsulating liposome (no OVA modification, FK-Lipo (5 µg/kg)), OVA-modified liposome (no encapsulation of tacrolimus, OVA-Lipo) and 5 µg/kg (weight) tacrolimus-encapsulating OVA-modified liposome (OVA-FK (5 µg/kg)) were used for administration. Each of the drugs was intravenously (tail vein) administered to each mouse group on Days 8, 10 and 12 following the sensitization day (Day 0) (total of three doses). Booster immunizations were carried out on Days 15 and 29 as described above. Sera were obtained from the mice on Days 22 and 36 by drawing blood from the fundus arteries with a hematocrit capillary tube (Terumo Corporation), which was left to stand still and then centrifuged. OVA-specific IgE in the mouse sera was quantified by ELISA.

### (6) Determination of IgE production by ELISA

Maxisorp 96-well plates (Nunc, Roskilde, Denmark) were coated through reaction with rat anti-mouse IgE monoclonal antibody (BD, Pharmingen) (2 µg/mL) at room temperature for 2 hours. Following two hours of blocking at room temperature with 1% gelatin-PBS, a triple dilution of the serum sample was added and incubated at 4°C overnight. A monoclonal antibody to ovalbumin (clone 2C6) (Acris Antibodies GmbH, Germany) was used as a calibration curve of OVA-specific IgE. OVA (10 µg/ml) biotinylated with an EX-LINK sulpho-NHS-LC biotinylation kit (PIERCE, Rockford, IL) was reacted at room temperature for an hour. Subsequently, peroxidase-conjugated streptavidin (Molecular Probes, Eugene, OR) was added for reaction at room temperature for an hour. Absorbance at 490 nm was determined using SIGMA FAST^{™} o-phenylendiamine dihydrochloride tablet sets as a substrate to give an antibody concentration.

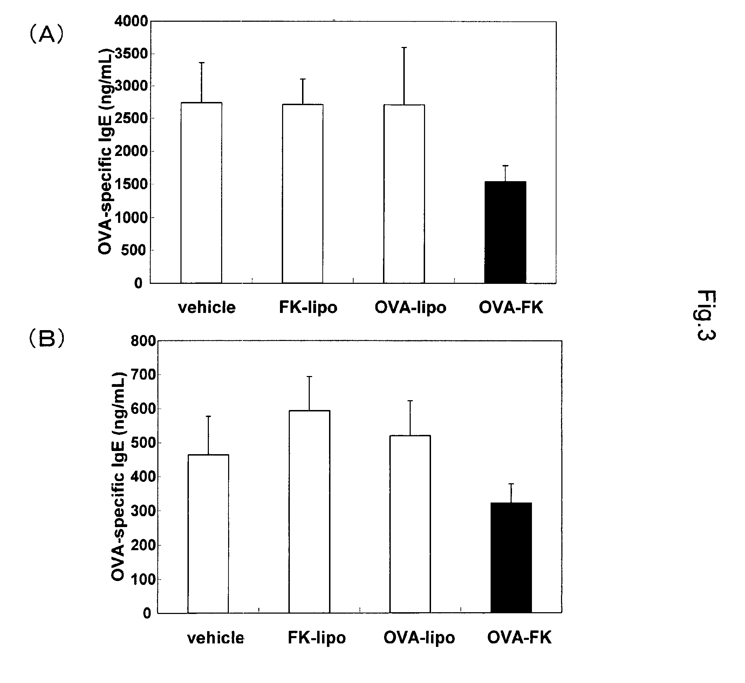

As a result, there was no significant difference in the weight loss of the mice between any test drug and Vehicle, indicating no side effects. Secondary, as to the influence on production of IgE, an immunoglobulin intimately related to various allergic reactions, the administration of OVA-FK (5 µg/kg) significantly decreased the IgE production as compared to the administrations of Vehicle, FK-Lipo (5 µg/kg) and OVA-Lipo (see Figures 3(A) and (B)). These results indicate that OVA-FK (5 µg/kg) has an effect of selectively and effectively suppressing IgE production unlike the drug merely encapsulating tacrolimus (Lipo-FK) or the drug merely modified with OVA (OVA-Lipo).

Based on the above data, RT-DDS was proven to be capable of selectively and effectively delivering a drug encapsulated in a liposome to a cell that recognizes the drug as a foreign substance. This proves the potential of RT-DDS to contribute to the improvement of therapeutic effects for allergic reaction to various foreign substances, for endogenous autoimmune diseases or the like by combining a new drug or an existing drug with RT-DDS.

## Claims

1. An immunomodulating agent comprising a lipid vesicle encapsulating a drug capable of suppressing or damaging an immunocyte that expresses or binds to an antibody, the lipid vesicle having an antigen for the antibody bound to the outer surface thereof.

2. The modulating agent according to Claim 1, wherein immunomodulation is immunosuppression.

3. A therapeutic agent for an immune disease comprising a lipid vesicle encapsulating a drug capable of suppressing or damaging an immunocyte that expresses or binds to an antibody, the lipid vesicle having an antigen for the antibody bound to the outer surface thereof.

4. The therapeutic agent according to Claim 3, wherein the immune disease is an allergic disease or an autoimmune disease.

5. A lipid vesicle encapsulating a drug capable of suppressing or damaging an immunocyte that expresses or binds to an antibody, the lipid vesicle having an antigen for the antibody bound to the outer surface thereof.
